# EUROPEAN PATENT APPLICATION

(11) **EP 3 437 567 A1**
(43) Date of publication of application: **06.02.2019**
(21) Application number: 16897122.4
(22) Date of filing: 28.12.2016
(51) Int. Cl.: A61B 10/00, A61B 5/00, G08B 25/04, G09B 19/00

(54) **INFORMATION PROCESSING DEVICE, INFORMATION PROCESSING METHOD AND PROGRAM**

(30) Priority: 31.03.2016 JP 2016072096
(71) Applicant: Sony Corporation, Tokyo 108-0075 (JP)
(72) Inventor: ASANO, Yasuharu, Tokyo 108-0075 (JP)
(74) Representative: MFG Patentanwälte Meyer-Wildhagen Meggle-Freund Gerhard PartG mbB
(86) International application number: PCT/JP2016/089131
(87) International publication number: WO 2017/168907

(57) **Abstract**

[Object] To provide an information processing device, an information processing method, and a program capable of recognizing a state of a brain function of the user through a natural interaction with the user.

[Solution] An information processing device including: an acquisition unit configured to acquire a response of a user to a question regarding personal information or action information of the user; a determination unit configured to determine true or false of the response; and a storage unit configured to store the question, the response, and a determination result in association with each other.

## Description

### Technical Field

The present disclosure relates to an information processing device, an information processing method, and a program.

### Background Art

Reflecting social circumstances of the falling birthrate and the aging population, in recent years, the number of elderly people who live alone (living-alone elderly people) is increasing. In accordance with the increase, there are growing needs of relatives such as children that live in remote locations, for the confirmation of safety and health states of living-alone elderly people.

In response to such needs, services for watching living-alone elderly people using information technology (IT) devices are provided. For example, such a service that an elderly person carries a wireless button, and when the button is pressed, a security agent rushes to the elderly person from a security company being under contract, and such a service that, when an elderly person uses an electrical pot, its usage history is delivered to a relative by e-mail are put into practical use.

Furthermore, in Patent Literature 1 described below, a safety confirmation service is disclosed. In the safety confirmation service, a touch panel display provided with buttons corresponding to actions, physical conditions, statuses, and demands of elderly people are prepared, and the safety is confirmed by the elderly people pressing the buttons by themselves. In addition to this, the safety is confirmed by receiving a meal delivery request in cooperation with a meal delivery service, and by a home-visit staff that visits a home for meal delivery manipulating the touch panel.

### Citation List

### Patent Literature

Patent Literature 1: JP 2015-146085A

### Disclosure of Invention

### Technical Problem

By the simple interaction with a user as described above, it is possible to recognize safety and a physical health state of the user to some extent, but it is difficult to discover, at an early date, a state regarding a cognition function of the user, that is to say, a sign of dementia.

According to the Ministry of Health, Labor and Welfare, early discovery is important as countermeasures for dementia, and in a case where a relative has an occasion of interacting with a living-alone elderly person only at times due to a distance or busyness, there is a possibility that the discovery of dementia is delayed, and the disease progresses. On the other hand, when a test for dementia as posted in http://test.ninchishouyobou-k.com/ or the like is performed, self-esteem (pride) of a living-alone elderly person is wounded in some cases.

In view of the foregoing, the present disclosure proposes an information processing device, an information processing method, and a program that can recognize a state of a brain function of a user though a natural interaction with the user.

### Solution to Problem

According to the present disclosure, there is proposed an information processing device including: an acquisition unit configured to acquire a response of a user to a question regarding personal information or action information of the user; a determination unit configured to determine true or false of the response; and a storage unit configured to store the question, the response, and a determination result in association with each other.

According to the present disclosure, there is proposed an information processing method including, by a processor: acquiring a response of a user to a question regarding personal information or action information of the user; determining true or false of the response; and storing, into a storage unit, the question, the response, and a determination result in association with each other.

According to the present disclosure, there is proposed a program for causing a computer to function as: an acquisition unit configured to acquire a response of a user to a question regarding personal information or action information of the user; a determination unit configured to determine true or false of the response; and a storage unit configured to store the question, the response, and a determination result in association with each other.

### Advantageous Effects of Invention

As described above, according to the present disclosure, it becomes possible to recognize a state of a brain function of a user though a natural interaction with the user.

Note that the effects described above are not necessarily limitative. With or in the place of the above effects, there may be achieved any one of the effects described in this specification or other effects that may be grasped from this specification.

### Brief Description of Drawings

[FIG. 1] FIG. 1 is a diagram describing an overview of an information processing device according to the present embodiment.
[FIG. 2] FIG. 2 is a block diagram illustrating an example of a configuration of the information processing device according to the present embodiment.
[FIG. 3] FIG. 3 is a diagram illustrating a functional configuration example of a control unit according to the present embodiment.
[FIG. 4] FIG. 4 is a diagram illustrating an example of data stored in a dialogue data storage unit according to the present embodiment.
[FIG. 5] FIG. 5 is a diagram illustrating an example of data stored in a user-related information storage unit according to the present embodiment.
[FIG. 6] FIG. 6 is a diagram describing a case of acquiring user-related information from a dialogue with a user according to the present embodiment.
[FIG. 7] FIG. 7 is a diagram illustrating an example of data stored in a speech information storage unit according to the present embodiment.
[FIG. 8] FIG. 8 is a flow chart illustrating a dialogue process according to the present embodiment.
[FIG. 9] FIG. 9 is a diagram illustrating an example of a dialogue according to the present embodiment.
[FIG. 10] FIG. 10 is a diagram illustrating an example of true-false determination to be performed on a user speech according to the present embodiment.
[FIG. 11] FIG. 11 is a flow chart illustrating an alert determination process according to the present embodiment.

### Mode(s) for Carrying Out the Invention

Hereinafter, (a) preferred embodiment(s) of the present disclosure will be described in detail with reference to the appended drawings. Note that, in this specification and the appended drawings, structural elements that have substantially the same function and structure are denoted with the same reference numerals, and repeated explanation of these structural elements is omitted.

In addition, the description will be given in the following order.
1. Overview of Information Processing Device According to Embodiment of Present Disclosure
2. Configuration of Information Processing Device
3. Operational Process
   3-1. Dialogue Process
   3-2. Alert Determination Process
4. Conclusion

### <<1. Overview of Information Processing Device According to Embodiment of Present Disclosure>>

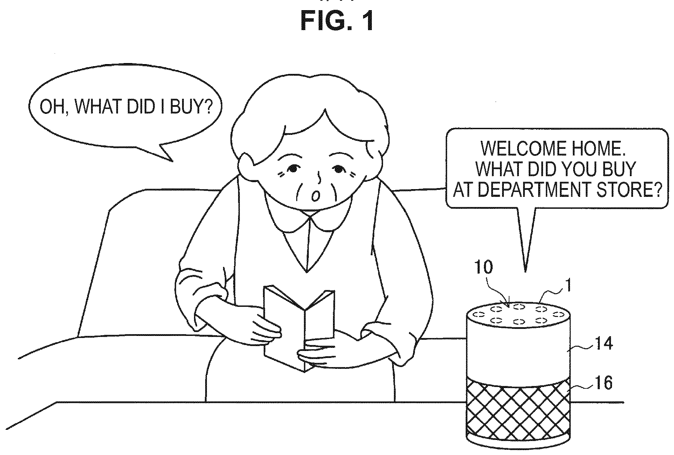

First of all, an overview of an information processing device according to an embodiment of the present disclosure will be described. FIG. 1 is a diagram describing an overview of an information processing device 1 according to the present embodiment. As illustrated in FIG. 1, the information processing device 1 according to the present embodiment includes a speech input unit 10 (e.g. microphone array) and a speech output unit 16, and has an agent function of implementing a voice dialogue with a user. The information processing device 1 acquires a speech voice of the user by the speech input unit 10, performs speech recognition and semantic analysis, generates response information to the speech of the user, and speaks (responds) to the user from the speech output unit 16. For example, in a case where an inquiry about tomorrow weather is received, the information processing device 1 accesses a weather information service via a network, acquires weather information of tomorrow, and conveys the weather information to the user.

Furthermore, the information processing device 1 may include an image output unit 14, and can display image information when making a response.

The information processing device 1 may be a standing home agent device as illustrated in FIG. 1, or may be a self-propelled home agent device (e.g. robot). In addition, the information processing device 1 may be a mobile terminal such as a smartphone, a tablet terminal, a mobile phone terminal, and a wearable terminal, or may be a device such as a personal computer, a game device, and a music player.

As described above, in these latter days in which the number of elderly people living alone (living-alone elderly people) is increasing, there are growing needs of relatives such as children that live in remote locations, for the confirmation of safety and health states of living-alone elderly people. By the conventional safety confirmation that is based on a simple interaction with a user (elderly person), it has been difficult to discover, at an early date, a state regarding a brain function of the user, specifically, a sign of dementia. Early discovery is important as countermeasures for dementia, and as for a living-alone elderly person who lives away from a relative, there is a possibility that the discovery of dementia is delayed, and the disease progresses.

In view of the foregoing, the information processing device 1 according to the present embodiment can recognize a state of a brain function of the user through a natural interaction (dialogue) with the user. Specifically, for example, in an interaction with the user, the information processing device 1 includes a question for confirming information related to the user, in a dialogue, and confirms whether a response of the user to the question is correct, thereby realizing early discovery of dementia of the user. The information related to the user can be acquired from the content of a usual dialogue with the user, and various types of information received from an external device or a network (sensor data, a captured image, a move history, a purchase history, a network usage history, an SNS post history, a view history, a device manipulation history, etc.).

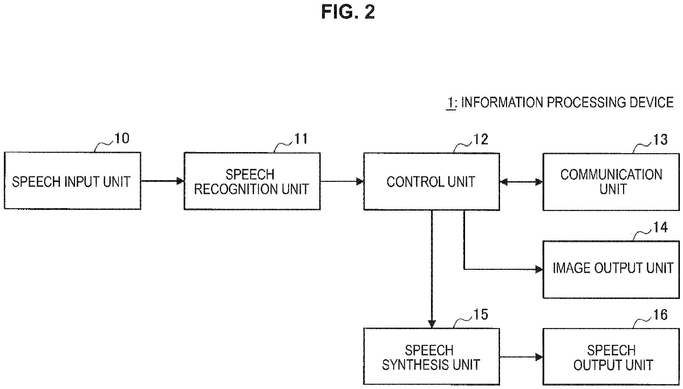

The overview of the information processing device 1 according to the present embodiment has been described above. Subsequently, a configuration of the information processing device 1 according to the present embodiment will be described with reference to FIG. 2.

### «2. Configuration»

FIG. 2 is a block diagram illustrating an example of a configuration of the information processing device 1 according to the present embodiment. As illustrated in FIG. 2, the information processing device 1 includes the speech input unit 10, a speech recognition unit 11, a control unit 12, a communication unit 13, the image output unit 14, a speech synthesis unit 15, and the speech output unit 16.

The speech input unit 10 collects a user voice and a surrounding environmental sound, and outputs a voice signal to the speech recognition unit 11. Specifically, the speech input unit 10 is implemented by a microphone, an amplifier, or the like. In addition, the speech input unit 10 may be implemented by a microphone array including a plurality of microphones.

The speech recognition unit 11 performs speech recognition on the voice signal output from the speech input unit 10, and converts the speech voice of the user into text. The speech data converted into text is output to the control unit 12.

The control unit 12 functions as an arithmetic processing unit and a control device, and controls overall operations in the information processing device 1 in accordance with various types of programs. For example, the control unit 12 is implemented by an electronic circuit such as a Central Processing Unit (CPU) and a microprocessor. In addition, the control unit 12 may include a Read Only Memory (ROM) that stores programs, calculation parameters, and the like that are to be used, and a Random Access Memory (RAM) that temporarily stores appropriately varying parameters and the like.

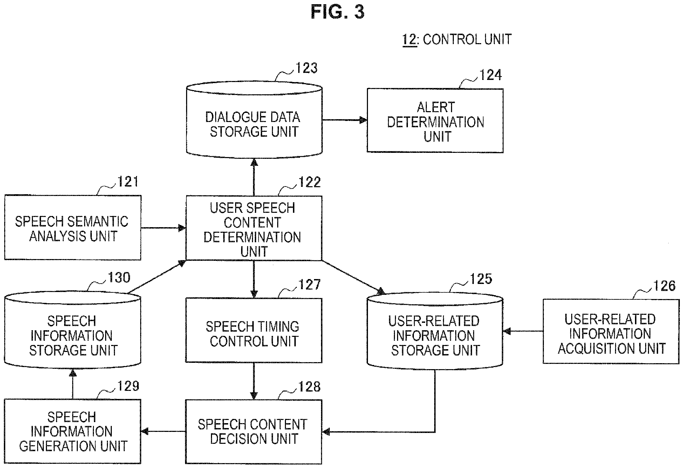

In addition, the control unit 12 according to the present embodiment generates speech information for responding to the user speech data (text information) output from the speech recognition unit 11, and autonomous speech information. The control unit 12 outputs the generated speech information to the image output unit 14 or the speech synthesis unit 15. The detailed configuration of the control unit 12 will be described later with reference to FIG. 3.

The communication unit 13 is a communication module that performs transmission and reception of data with another device in a wired/wireless manner. The communication unit 13 performs wireless communication with an external device directly or via a network access point, using a system of a wired Local Area Network (LAN), a wireless LAN, Wireless Fidelity (Wi-Fi, registered trademark), infrared communication, Bluetooth (registered trademark), and near field/noncontact communication, for example,

The communication unit 13 according to the present embodiment receives various types of information from a camera, a user terminal, and various sensors, for example. The various sensors may be provided on a user terminal, may be provided on a wearable terminal worn by the user, or may be installed on a door or a sofa of a room, a passage way, or the like. As the various sensors, for example, a gyro sensor, an acceleration sensor, a direction sensor, a positioning unit, a biosensor, and the like are assumed.

The image output unit 14 is implemented by, for example, a liquid crystal display (LCD) device, an Organic Light Emitting Diode (OLED) device, or the like. The image output unit 14 displays image information output from the control unit 12, to the user.

The speech synthesis unit 15 converts the speech information (text) output from the control unit 12, into voice data (into voice), and outputs the voice data to the speech output unit 16.

The speech output unit 16 outputs the voice data output from the speech synthesis unit 15, to the user. Specifically, the speech output unit 16 is implemented by a speaker, an amplifier, or the like.

### (Detailed Configuration of Control Unit 12)

Subsequently, the detailed configuration of the control unit 12 according to the present embodiment will be described with reference to FIG. 3. FIG. 3 is a diagram illustrating a functional configuration example of the control unit 12 according to the present embodiment.

As illustrated in FIG. 3, the control unit 12 according to the present embodiment functions as a speech semantic analysis unit 121, a user speech content determination unit 122, a dialogue data storage unit 123, an alert determination unit 124, a user-related information storage unit 125, a user-related information acquisition unit 126, a speech timing control unit 127, a speech content decision unit 128, a speech information generation unit 129, and a speech information storage unit 130.

The speech semantic analysis unit 121 applies a so-called natural language process to the speech data (text) input from the speech recognition unit 11, and performs the extraction of a keyword in the speech, the estimation of speech intent of the user, and the like. A speech analysis result is output to the user speech content determination unit.

The user speech content determination unit 122 performs two processes in accordance with the speech analysis result output from the speech semantic interpretation unit. The first process is a process of determining whether user-related information is included in the speech analysis result, and in a case where user-related information is included, registering content of the user-related information into the user-related information storage unit 125. For example, personal information or an action history that is related to the user or a family of the user, such as what the user ate for dinner, where the user went, what the user bought, or the name or birthday of a grandchild, is extracted from the speech analysis result, and registered into the user-related information storage unit 125. By continuously performing this registration process, the user-related information can be updated.

The second process is a process of determining true or false of speech content of the user on the basis of the speech analysis result, and immediate speech content of a system side (i.e. the information processing device 1 side) that is stored in the speech information storage unit 130, which will be described later. The determination result is stored into the dialogue data storage unit 123 in association with the speech content (question) of the system side and an analysis result (response) of the user speech. More specifically, the user speech content determination unit 122 determines whether the response content of the user to a question of the system side has no problem, with reference to the user-related information stored in the user-related information storage unit 125. The determination result can be classified into several patterns prepared in advance. For example, the determination result is classified in the following manner.
CORRECT: no problem
FORGET: not remember
WRONG_MEMORY: wrongly memorize
INCONSISTENT: inconsistent with previous speech content of the user
NOT_CLASSIFIABLE: indeterminable

Note that, although the user-related information stored in the user-related information storage unit 125 is presupposed to be correct data, in a case where an answer of the user to a question regarding information acquired from a dialogue with the user is wrong, the answer is determined to be "INCONSISTENT: inconsistent with previous speech content of the user".

In addition, the user speech content determination unit 122 may determine what type of information is forgotten to what extent, and in addition, in a case where a question regarding a history of an action is asked, may perform determination considering the lapse of time since the action has been performed, in addition to the true-false determination as described above.

In addition, a level at which determination is made to be "CORRECT: no problem" varies depending on the granularity of information registered in the speech information storage unit 130, but if a degree of variations (ambiguity) in the user speech content is a certain degree, the user speech content can be complemented with data acquired from an external server, and determined to be "CORRECT: no problem". For example, in a case where information indicating "outgo destination: xx department store @ A town" is registered in the speech information storage unit 130, in a case where an answer of the user to a question of the system side that indicates "where did you go?" is "C shop in A town", the user speech content determination unit 122 searches a network for information indicating where in the A town the 'C shop' exists. Then, in a case where information indicating that the 'C shop' is located in the 'xx department store' is obtained, the user speech content determination unit 122 can determine the answer of the user to be "CORRECT: no problem".

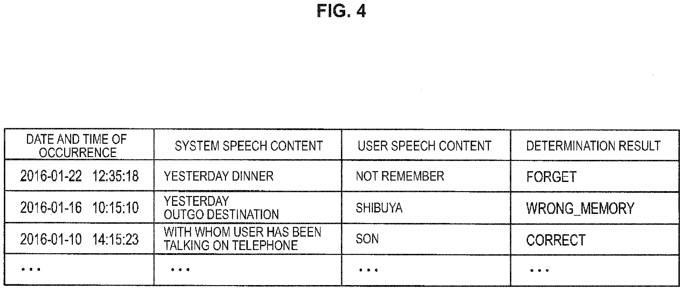

The dialogue data storage unit 123 stores information (system side speech content and user speech content) used in the determination in the user speech content determination unit 122, and the determination result, in association with each other. Here, an example of data in the dialogue data storage unit 123 is illustrated in FIG. 4. As illustrated in the drawing, date and time of occurrence, system speech content, user speech content, and a determination result are held. The date and time of occurrence is date and time at which a dialogue (interaction) of a question and a response has been performed. In addition, the system speech content is a confirmation item asked to the user ("yesterday dinner", "yesterday outgo destination", "with whom the user has been talking on a telephone", etc.), for example. In addition, the user speech content is response content of the user ("not remember", "Shibuya", "son", etc.). The determination result indicates to which of the several patterns classified in advance as described above, the response content corresponds, for example.

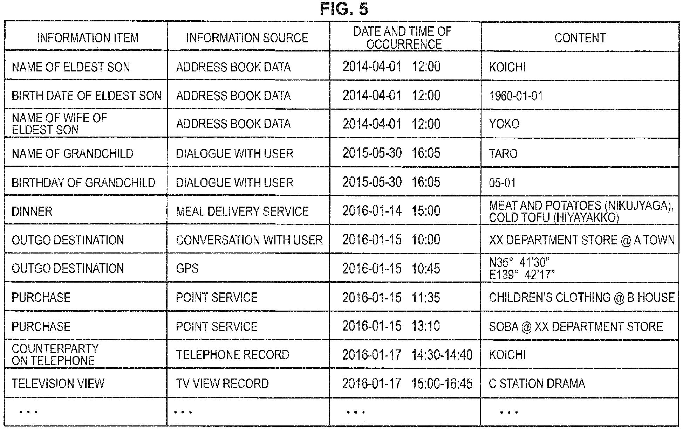

The user-related information storage unit 125 stores personal information of the user (e.g. name, age, and birthday of the user, name, age, and birthday of a relative, etc.) or an action history of the user (content of meals, outgo history, view history, etc.). In this specification, action information includes at least one of an action history, an action plan, and an operation during an action (in the following description, the action history is used as the action information). Here, an example of data stored in the user-related information storage unit 125 is illustrated in FIG. 5. As illustrated in the drawing, the user-related information has a data configuration in which an information item, an information source, date and time of occurrence, and content are associated with each other.

The information item is a classification of stored information, and for example, "a name of an eldest son", "birth date of an eldest son", "dinner", "outgo destination", "purchase", and the like are assumed as illustrated in FIG. 5.

The information source indicates from where the information has been acquired, and there are information obtained from a user speech, and information obtained by the user-related information acquisition unit 126. The information obtained from a user speech is information to be registered into the user-related information storage unit 125 when the information is determined to be user-related information by the user speech content determination unit 122 on the basis of the speech analysis result obtained by the speech semantic analysis unit 121. In this case, the information source becomes "a dialogue with the user" as illustrated in FIG. 5. In addition, the information obtained by the user-related information acquisition unit 126 is specifically information obtained via the communication unit 13 from a user terminal or various types of servers on a network, or a peripheral device. For example, "address book data" is obtained from the user terminal or the network. In addition, "GPS" is position information detected by a position sensor such as the Global Positioning System (GPS) that is provided on the user terminal. In addition, a "meal delivery service" and a "point service" are examples of the external servers, and are obtained from the network. In addition, a "telephone record" is obtained from the user terminal or a land phone for domestic use. In addition, a "TV view record" is obtained from a TV connected in a wireless/wired manner.

The date and time of occurrence is date and time at which the information has been acquired (recorded), or date and time at which an event indicated by the information has occurred. The content is content of the information.

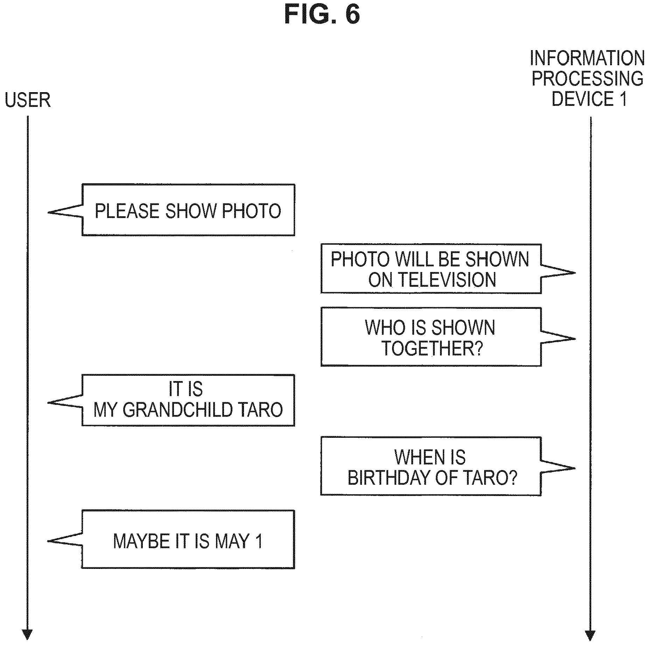

Here, the description will be given of a case of acquiring user-related information from a dialogue with the user, with reference to FIG. 6. FIG. 6 illustrates contents of speeches performed by the user and the information processing device 1 (system), in chronological order. As illustrated in the drawing, for example, in response to a photo display request from the user that indicates "please show a photo", the information processing device 1 answers that "a photo will be shown on a television", transmits image information to the television via the communication unit 13, and displays the photo on a television screen. At this time, the information processing device 1 asks a question regarding the displayed photo. For example, in a case where it is identified by image analysis that two people are included in the photo and one of them is the user, a topic regarding a person shown in the photo together with the user is presented to the user, and information regarding the person is acquired on the basis of a response of the user. In the example illustrated in FIG. 6, for example, a question indicating "who is shown together?" is asked, and from an answer of the user that indicates "it is my grandchild Taro", user-related information indicating "a name of a grandchild: Taro" is acquired. Furthermore, a question indicating "when is the birthday of Taro?" is asked, and from an answer of the user that indicates "maybe it is May 1", user-related information indicating "a birthday of a grandchild: May 1" is acquired.

The user-related information described above is used when a question to the user is decided by the speech content decision unit 128, which will be described later, and when true or false of a response of the user is determined by the user speech content determination unit 122.

The user-related information acquisition unit 126 acquires, via the communication unit 13, user-related information from various types of servers on a network, a user terminal, a wearable device, a peripheral device, or the like. By acquiring user-related information from various types of servers on a network, cooperation with various types of external services is enabled. For example, the user-related information acquisition unit 126 can access a server provided by a meal delivery service company for elderly people that is under contract with the user, acquire everyday menu information, and register information indicating what type of meal the user ate on a specific day, into the user-related information storage unit 125 as user-related information.

In a similar manner, by cooperation with a goods selling service used by the user, information regarding a product recently purchased by the user can be acquired, and by cooperation with a moving image delivery or music delivery service, information regarding a film recently viewed by the user or music recently listened to by the user can also be acquired.

In addition, from information of a position sensor of a user terminal or a wearable device, the user-related information acquisition unit 126 can acquire information regarding an outgo destination of the user. Specifically, the user-related information acquisition unit 126 can identify a location where the user exists, on the basis of latitude-longitude information obtained from the position sensor, and location information obtained from a Geographic Information Systems (GIS) service, and registers the information into the user-related information storage unit 125. The position sensor mounted on the user terminal or the wearable device is implemented by a Global Positioning System (GPS) positioning unit, for example, and detects a position at which the position sensor exists, by receiving radio waves from a GPS satellite. In addition, the position sensor may detect the position by, for example, Wi-Fi (registered trademark), Bluetooth (registered trademark), transmission and reception with a mobile phone, a personal handyphone system (PHS), or a smartphone, near field communication, or the like, aside from the GPS.

The alert determination unit 124 checks data stored in the dialogue data storage unit 123, and reports, as necessary, alert information to a predetermined contact name such as a relative and a primary care doctor, via a network from the communication unit 13. For example, on the basis of determination results stored in the dialogue data storage unit 123, the alert determination unit 124 may report alert information in a case where problematic determination results exceed a certain rate. In addition, the alert determination unit 124 may perform a statistical process on the basis of the determination results, and report alert information in a case where a calculation result satisfies a predetermined condition.

The speech timing control unit 127 controls a timing of a speech to the user. For example, aside from performing control so as to make a response when being spoken to from the user, the speech timing control unit 127 performs control so as to autonomously speak when detecting wake-up or return home of the user from information of a camera, a human sensor, or the like that is connected via a network.

The speech content decision unit 128 decides content to be spoken to the user. For example, in a case where a demand of some sort is received from the user, such as a case where an inquiry about tomorrow weather is received, for example, the speech content decision unit 128 accesses a weather information server via a network, and decides acquired tomorrow weather information as speech content (response). In addition, the speech content decision unit 128 may decide, as speech content, a question for confirming whether the user remembers content appropriately selected from information registered in the user-related information storage unit 125.

A process performed by the speech content decision unit 128 can be efficiently decided by preparing patterns of speech content in advance. For example, the speech content decision unit 128 decides the following speech contents.
- Inform (Item, Date, Value): make a response to inform the user that "a value of Item" on "a day of Date" is "Value".
- Ask (Item, Date): ask the user about "a value of Item" on "a day of Date".

On the basis of the speech content decided by the speech content decision unit 128, the speech information generation unit 129 generates speech information to be actually presented to the user. For example, if the speech content decided by the speech content decision unit 128 is "Inform (Weather, Tomorrow, Fine)", the speech information generation unit 129 generates a response sentence indicating that "tomorrow weather is fine". In addition, if the speech content decided by the speech content decision unit 128 is "Ask (visit_place, Yesterday)", the speech information generation unit 129 generates a question sentence asking that "where did you go yesterday?". The speech information generated by the speech information generation unit 129 is output to the image output unit 14 or the speech synthesis unit 15. In a case where the speech information is output to the image output unit 14, the speech information (text) is displayed on a screen or projected onto a wall or the like. In addition, in a case where the speech information is output to the speech synthesis unit 15, the speech information (text) is converted into a voice, and reproduced from the speech output unit 16. In addition, the speech information may be transmitted from the communication unit 13 to a peripheral display device, a speech output device, a user terminal, a wearable device, and the like that are connecting thereto, and may be presented to the user from these external devices.

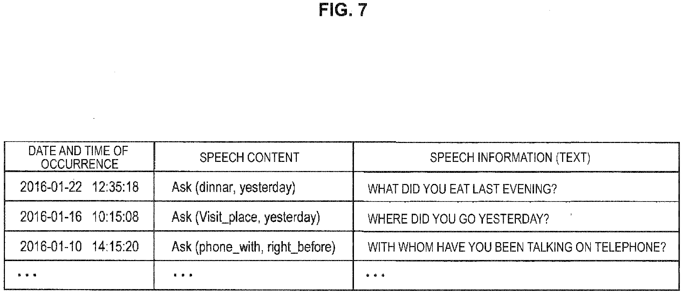

The speech information storage unit 130 stores the speech content decided by the speech content decision unit 128, and the speech information generated by the speech information generation unit 129. Here, an example of data stored in the speech information storage unit 130 is illustrated in FIG. 7. As illustrated in the drawing, the data has a data configuration in which speech date and time, speech content, and speech information (text) of the system side are associated with each other.

The configuration of the information processing device 1 according to the present embodiment has been specifically described above. Note that, the configuration of the information processing device 1 according to the present embodiment is not limited to the examples illustrated in FIGS. 2 and 3. For example, a part of the configurations of the information processing device 1 may be provided in an external device (including a server on a cloud) connecting thereto via the communication unit 13. In addition, the information processing device 1 may include a human sensor and a camera.

### «3. Operation»

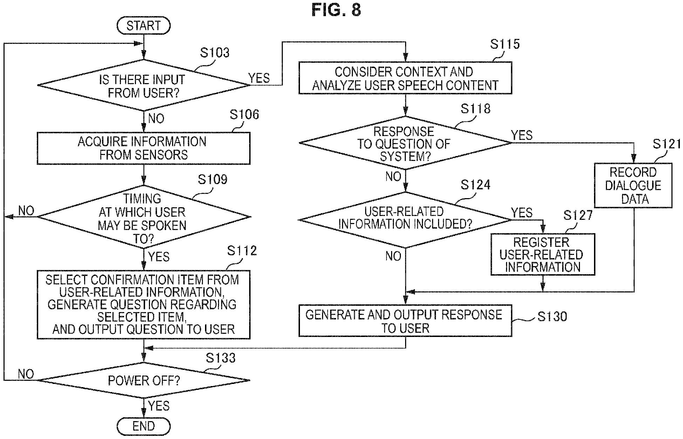

Subsequently, an operational process according to the present embodiment will be described with reference to FIGS. 8 to 9.

### <3-1. Dialogue Process>

FIG. 8 is a flow chart illustrating a dialogue process according to the present embodiment. The dialogue process according to the present embodiment is executed by a system (application program) starting up in the information processing device 1.

As illustrated in FIG. 8, first of all, in a case where there is an input (speech) from the user (step S103/Yes), the control unit 12 of the information processing device 1 considers a context, and analyzes user speech content (step S115). Considering the context means considering whether the user speech is a response to a question from the information processing device 1 (system).

In a case where the user speech is not a response to a question from the system (step S118/No), the user speech content determination unit 122 determines whether user-related information is included in the speech content (step S124).

In a case where user-related information is included (step S124/Yes), the user speech content determination unit 122 registers the user-related information into the user-related information storage unit 125 (step S127).

On the other hand, in a case where the user speech is a response to a question from the system (step S118/Yes), the user speech content determination unit 122 determines whether the response is appropriate for the question, and stores, into the dialogue data storage unit 123, the determination result, the user speech (response), and immediate system speech (question) in association with each other, as dialogue data (step S121).

After that, the control unit 12 decides response content to the speech of the user by the speech content decision unit 128, generates speech information by the speech information generation unit 129, and presents the speech information to the user by speech output or image output (step S130).

In addition, in a case where there is no input from the user (step S103/No), the information processing device 1 acquires information from various types of sensors (step S106). For example, the information processing device 1 receives, via the communication unit 13, information from a human sensor provided in a living room, a sensor interlocked with power ON/OFF of a television, and the like.

Next, the speech timing control unit 127 determines whether it is a timing at which the user may be spoken to (step S109). Examples of appropriate timings of speaking to the user include a timing at which the user returns to a home (state is switched from an absence state to a presence state) that is determined on the basis of data acquired from a human sensor, and a timing at which the user turns off a television that is determined on the basis of data acquired from a sensor interlocked with power ON/OFF of the television. In addition, the examples of appropriate timings of speaking to the user also include a timing at which the user ends a telephone call that is determined on the basis of data acquired from a sensor interlocked with a telephone device, and the like.

In a case where the timing is determined to be not appropriate as a timing of speaking to the user (step S109/No), the processing returns to step S103 described above.

On the other hand, in a case where the timing is determined to be appropriate as a timing of speaking to the user (step S109/Yes), the speech content decision unit 128 selects an item for making a confirmation to the user, from user-related information registered in the user-related information storage unit 125, and decides a question (speech content) regarding the selected item. Then, speech information (question sentence) is generated by the speech information generation unit 129, and the speech information is presented to the user by image output or speech output (step S112).

Here, examples of questions of confirmation items for the user include questions as described below. The questions according to the present embodiment may be decided by referring to items presented in "guideline for dementia early discovery" (http://www.alzheimer.or.jp/?page_id=2196) proposed by the Alzheimer's Association Japan, for example, and the like.
- Inquire with whom the user had been talking, after a telephone has been hung up. The true-false determination can be performed by acquiring a counterparty on a telephone from address book data registered in a telephone device.
- Inquire about performers or content after viewing of a television program ends. The true-false determination can be performed by acquiring information regarding content of a television, from a view history of the television and an electronic program guide.
- Inquire about a day of the week or a month.
- Display a photo of an acquaintance on a display, and inquire who is shown on the display.
- Inquire where the user lives, and in addition, inquire what type of job the user does.
- Perform a game (game or the like of saying names of vegetables, names of animals, and the like).

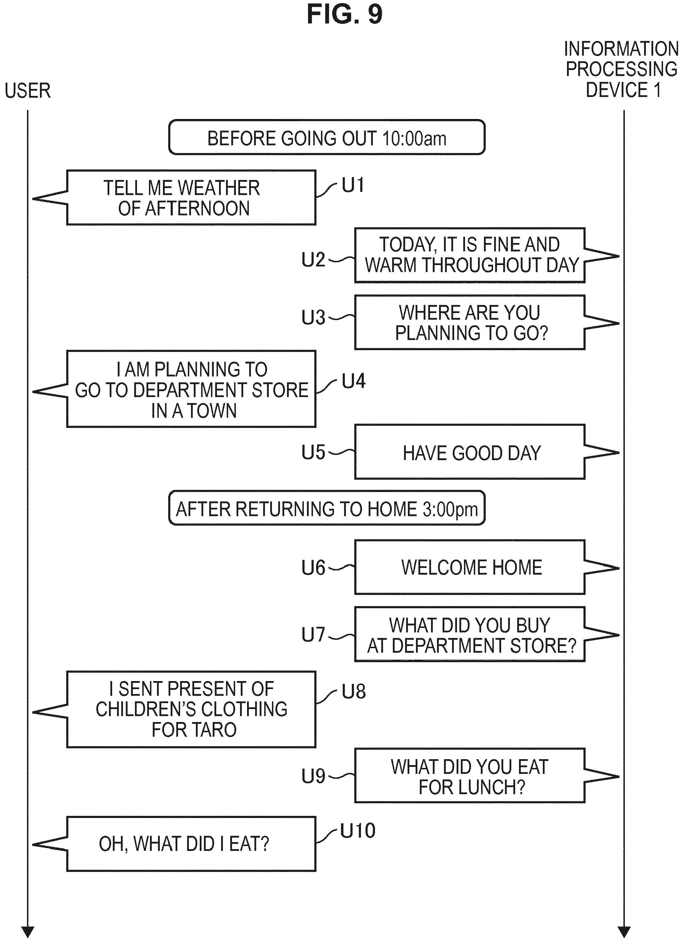

In addition, the information processing device 1 according to the present embodiment may frequently perform a dialogue of providing convenience or amusement to the user, without always performing a speech (question) of confirming user-related information. The speech of confirming user-related information is moderately mixed into such dialogues, and a speech timing is controlled such that the user does not become conscious of undergoing the test of dementia. Here, an example of a dialogue according to the present embodiment is illustrated in FIG. 9. FIG. 9 illustrates contents of speeches performed by the user and the information processing device 1 (system), in chronological order.

As illustrated in the drawing, for example, when the user performs a speech U1 of inquiring about weather forecast of this afternoon, to the information processing device 1, the information processing device 1 acquires weather forecast information from a network, and performs a speech U2 of making a response. Furthermore, because the user confirms weather forecast, the information processing device 1 estimates that it is a timing at which the user is to go out, and performs a speech U3 of inquiring where the user is planning to go. In response to this, when the user makes such an answer that the user is planning to go to a department store in an A town, the information processing device 1 registers the answer as user-related information. At this time, the user speech content determination unit 122 of the information processing device 1 may make an inquiry to a calendar application or the like, to confirm a schedule of the user, and perform matching. The information processing device 1 lastly performs a greeting speech U5 "have a good day", and ends a series of dialogue controls.

Next, at a timing at which the user returns to a home from the outside, the information processing device 1 performs a greeting speech U6 "welcome home", and progresses a conversation with a topic regarding the department store because information indicating that the user is planning to go to the department store has been obtained from the dialogue with the user before the user goes out. For example, in a case where information regarding a product bought by the user at the department store in the A town is acquired from an external point service management server, a card company server, or the like, and is registered in the user-related information storage unit 125, an appropriate item is selected from shopping information, and the selected item is asked to the user. For example, a speech U7 of inquiring what the user bought at the department store, and a speech U9 of inquiring what the user ate for lunch are performed. Then, the information processing device 1 performs true-false determination of speeches U8 and U10 of responses from the user to these questions, by the user speech content determination unit 122, and stores determination results into the dialogue data storage unit 123.

In this manner, in the present embodiment, because questions of confirmation items are asked to the user in a flow of a natural conversation, a state of a cognition function can be recognized without causing the user to become conscious of that the questions are question items for dementia early discovery.

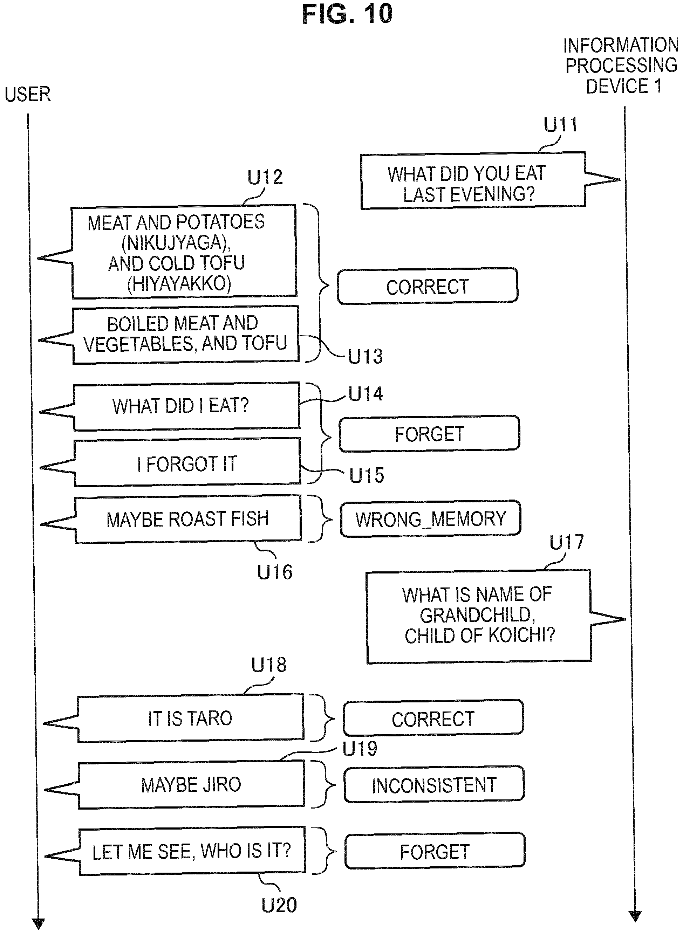

After that, an example of true-false determination to be performed on speech content of the user will be described with reference to FIG. 10. FIG. 10 illustrates contents of speeches performed by the user and the information processing device 1 (system), in chronological order. As illustrated in the drawing, the information processing device 1 performs a speech U11 "what did you eat last evening?" for asking menu that the user ate last evening, and when the user performs a speech U12, U13, U14, U15, or U16 of a response, the information processing device 1 determines, by the user speech content determination unit 122, true or false of the speech content with reference to user-related information stored in the user-related information storage unit 125.

Specifically, for example, in a case where the response of the user indicates that the user does not remember or in a case where a specific answer to the question is not obtained, such as the speech U14 "what did I eat?" or the speech U15 "I forgot it", the user speech content determination unit 122 determines the response to be "FORGET".

In addition, in a case where a specific answer is obtained, the user speech content determination unit 122 determines the response to be any of "CORRECT", "WRONG_MEMORY", and "INCONSISTENT" The determination process is performed while performing matching with user-related information stored in the user-related information storage unit 125, but in speeches of the user that are represented by natural language, because there are a plurality of wordings for representing the same event, only by the matching with user-related information, a determination range of "CORRECT" becomes extremely narrow. For example, in a case where the user makes a response "boiled meat and vegetables, and tofu" (speech U13) to the question of confirming menu that the user ate last evening, but "meat and potatoes (Nikujyaga), cold tofu (Hiyayakko)" is registered in the user-related information storage unit 125, the user-related information and the response content do not match, and the response is determined to be "WRONG_MEMORY". Nevertheless, by acquiring recipe information from a predetermined server (e.g. a server of a meal delivery service that provides dinner to the user), and utilizing information indicating that ingredients of "meat and potatoes (Nikujyaga)" are pork, potatoes, carrots, and onions, and in addition, an ingredient of "cold tofu (Hiyayakko)" is tofu, as so-called ontology, the information processing device 1 can determine that the above response content of the user is correct. The recipe information is desirably acquired from the server of the meal delivery service that actually provides dinner to the user, but the present embodiment is not limited to this, and the recipe information may be acquired from a general recipe information site.

Note that, in a case where the user makes a response "meat and potatoes (Nikujyaga), and cold tofu (Hiyayakko)" (speech U12), because the response matches the registered information, the response is determined to be "CORRECT".

In a similar manner, for example, as for an outgo destination, in a case where the user has been to a B house (shop name) at a xx department store in the A town", in a case where the user makes a response "A town", "xx department store", or "B house" to a question "where did you go?" of the information processing device 1, all of the responses are determined to be correct ("CORRECT"). As for the information regarding an outgo destination, for example, information that can be acquired from the Geographic Information System (GIS) using latitude and longitude at an outgo destination that are acquired by the GPS of the user terminal, a purchase history acquired from a server of a point service or the like, and the like are used as ontology information.

After that, in a case where a specific answer has been obtained, but the answer is not correct, if registered user-related information is information that is based on a previous dialogue with the user, the user speech content determination unit 122 determines the answer to be "INCONSISTENT", and if the registered user-related information is information that is based on information other than dialogues with the user, the user speech content determination unit 122 determines the answer to be "WRONG_MEMORY".

The user-related information that is based on information other than dialogues with the user is information mainly obtained from an external server or various types of sensors, and is highly likely to be true, and a reason why the response of the user fails to be determined to be "CORRECT" is mainly assumed to be lapse of memory of the user, and the response is determined to be "WRONG_MEMORY". For example, in the example illustrated in FIG. 10, in a case where information indicating that dinner is "meat and potatoes (Nikujyaga), cold tofu (Hiyayakko)" has been obtained from the meal delivery service server, in a case where a response of the user is "maybe roast fish" (speech U16), the user speech content determination unit 122 determines the response to be "WRONG_MEMORY".

On the other hand, as for the user-related information that is based on a dialogue with the user, because it is difficult to determine which of the previous dialogue with the user and the current speech of the user is true, the user speech content determination unit 122 determines the response to be "INCONSISTENT" indicating mere inconsistency with previous statement.

For example, in a case where user-related information "grandchild name: Taro" that is based on a dialogue with the user is registered in the user-related information storage unit 125, in a case where the information processing device 1 asks "what is the name of a grandchild, a child of Koichi?" (speech U17), and the user makes a correct response "it is Taro" (speech U18), as illustrated in FIG. 10, the response is determined to be "CORRECT". In addition, in a case where the user makes a wrong response "maybe Jiro" (speech U19), the response is determined to be "INCONSISTENT". In addition, in a case where the user says "let me see, who is it?", and does not make a specific answer, the response is determined to be "FORGET".

Note that, even in a case where a response of the user is determined to be incorrect ("FORGET", "WRONG_MEMORY", or "INCONSISTENT"), the information processing device 1 naturally continues a dialogue by saying "I see" or the like, without pointing out the error, thereby preventing the user from becoming aware of that the question is a test of a cognitive mechanism.

The above-described dialogue process in steps S103 to S130 illustrated in FIG. 8 is repeated until power of the information processing device 1 is turned off, and the system enters an end state (step S133).

### <3-2. Alert Determination Process>

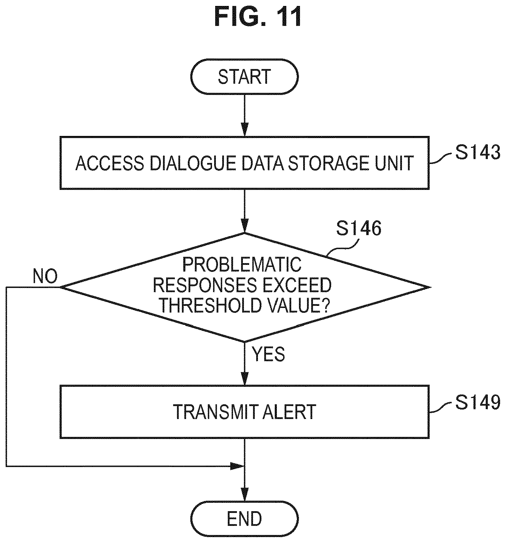

Next, an operational process performed by the alert determination unit 124 according to the present embodiment will be described with reference to FIG. 11. FIG. 11 is a flow chart illustrating an alert determination process according to the present embodiment. The alert determination process illustrated in FIG. 11 is executed when a predetermined condition is satisfied, for example, at a determined time of each day (each week), or at every certain period of time.

As illustrated in FIG. 11, first of all, the alert determination unit 124 accesses the dialogue data storage unit 123, and acquires a response history of the user to questions from the information processing device 1, and determination results thereof (step S143).

After that, the alert determination unit 124 compiles determination results, obtains a rate of problematic responses (e.g. responses determined to be "FORGET", "WRONG_MEMORY", or "INCONSISTENT", etc.), and compares the obtained rate with a preset threshold value (step S146). At this time, the alert determination unit 124 can not only obtain a rate of problematic responses in a certain period of time and perform comparison with a threshold value, but also compile temporal variations in rate of problematic responses by shifting a period of time in which compiling is performed, and make comparison with another threshold value.

Then, in a case where the rate of problematic responses exceeds the threshold value (step S146/Yes), the alert determination unit 124 transmits alert (e.g. alert including a report about a dementia sign of an elderly person) to a pre-registered contact name (e.g. kindred, primary care doctor, etc.) (step S149).

Lastly, in the present embodiment, an interaction between the user and the system by voice has been mainly described, but the interaction is not limited to this. For example, the interaction can be implemented by using a display equipped with a touch panel, and can be implemented by using inputs performed by a display and a keyboard.

### «4. Conclusion»

As described above, in the information processing device 1 according to an embodiment of the present disclosure, a state of a brain function of the user can be recognized through a natural interaction with the user.

Specifically, according to the present embodiment, the user can receive a check for a decline in a cognition function, through talking mixed in an interaction (dialogue), while receiving convenience and amusement provided by the agent function. This prevents the user being a living-alone elderly person, from feeling bothersome by taking the trouble of undergoing a test, and in addition, enables a relative of the user to discover a decline in a cognition function of the living-alone elderly person at an early date, so that appropriate treatment can be performed by a doctor.

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

For example, a computer program for fulfilling a function of the information processing device 1 can also be created in hardware such as a CPU, a ROM, and a RAM that is built-in the above-described information processing device 1. In addition, a computer-readable storage medium storing the computer program is also provided.

Note that, a dialogue performed between the information processing device 1 according to the present embodiment and the user is not limited to a voice dialogue, and may be gesture (sign language, body language signal, hand gesture) or text (chat). In this case, for example, an interaction is implemented via a display equipped with a touch panel, inputs performed by a display and a keyboard, and the like.

Further, the effects described in this specification are merely illustrative or exemplified effects, and are not limitative. That is, with or in the place of the above effects, the technology according to the present disclosure may achieve other effects that are clear to those skilled in the art from the description of this specification.

Additionally, the present technology may also be configured as below.
(1) An information processing device including:
   an acquisition unit configured to acquire a response of a user to a question regarding personal information or action information of the user;
   a determination unit configured to determine true or false of the response; and
   a storage unit configured to store the question, the response, and a determination result in association with each other.
(2) The information processing device according to (1), further including:
   a transmission unit configured to transmit a determination result stored in the storage unit, to an external device.
(3) The information processing device according to (1) or (2), further including:
   a generation unit configured to generate the question for confirming whether the user memorizes content of user-related information at least including personal information or action information of the user; and
   an output unit configured to output the question.
(4) The information processing device according to (3), in which the generation unit generates a natural question corresponding to a flow of a dialogue with the user, or to an action of the user.
(5) The information processing device according to (3) or (4), in which the determination unit determines true or false of the response with reference to the user-related information.
(6) The information processing device according to (5), in which, in a case where a question regarding a history of an action is asked, the determination unit performs determination considering a lapse of time since the action has been performed.
(7) The information processing device according to (5) or (6), in which the determination unit determines what type of information is forgotten to what extent, in addition to true-false determination.
(8) The information processing device according to any one of (3) to (7), in which the user-related information at least includes personal information regarding the user, or an action history of the user.
(9) The information processing device according to (8), in which the action history is extracted from content of a dialogue with the user, sensor data, a captured image, a move history, a purchase history, a network usage history, an SNS post history, a view history, or a device manipulation history.
(10) The information processing device according to any one of (1) to (9), further including:
   an alert determination unit configured to perform a statistical process on a basis of a determination result stored in the storage unit, and to determine whether to perform alert to an external device, in accordance with a calculation result.
(11) The information processing device according to (10), further including:
   a transmission unit configured to transmit alert to the external device in a case where the calculation result satisfies a predetermined condition.
(12) The information processing device according to (10) or (11), in which the alert is alert regarding a dementia sign of an elderly person.
(13) An information processing method including, by a processor:
   acquiring a response of a user to a question regarding personal information or action information of the user;
   determining true or false of the response; and
   storing, into a storage unit, the question, the response, and a determination result in association with each other.
(14) A program for causing a computer to function as:
   an acquisition unit configured to acquire a response of a user to a question regarding personal information or action information of the user;
   a determination unit configured to determine true or false of the response; and
   a storage unit configured to store the question, the response, and a determination result in association with each other.

### Reference Signs List

- 1: information processing device
- 10: speech input unit
- 11: speech recognition unit
- 12: control unit
- 13: communication unit
- 14: image output unit
- 15: speech synthesis unit
- 16: speech output unit
- 121: speech semantic analysis unit
- 122: user speech content determination unit
- 123: dialogue data storage unit
- 124: alert determination unit
- 125: user-related information storage unit
- 126: user-related information acquisition unit
- 127: speech timing control unit
- 128: speech content decision unit
- 129: speech information generation unit
- 130: speech information storage unit

## Claims

1. An information processing device comprising:
an acquisition unit configured to acquire a response of a user to a question regarding personal information or action information of the user;
a determination unit configured to determine true or false of the response; and
a storage unit configured to store the question, the response, and a determination result in association with each other.

2. The information processing device according to claim 1, further comprising:
a transmission unit configured to transmit a determination result stored in the storage unit, to an external device.

3. The information processing device according to claim 1, further comprising:
a generation unit configured to generate the question for confirming whether the user memorizes content of user-related information at least including personal information or action information of the user; and
an output unit configured to output the question.

4. The information processing device according to claim 3, wherein the generation unit generates a natural question corresponding to a flow of a dialogue with the user, or to an action of the user.

5. The information processing device according to claim 3, wherein the determination unit determines true or false of the response with reference to the user-related information.

6. The information processing device according to claim 5, wherein, in a case where a question regarding a history of an action is asked, the determination unit performs determination considering a lapse of time since the action has been performed.

7. The information processing device according to claim 5, wherein the determination unit determines what type of information is forgotten to what extent, in addition to true-false determination.

8. The information processing device according to claim 3, wherein the user-related information at least includes personal information regarding the user, or an action history of the user.

9. The information processing device according to claim 8, wherein the action history is extracted from content of a dialogue with the user, sensor data, a captured image, a move history, a purchase history, a network usage history, an SNS post history, a view history, or a device manipulation history.

10. The information processing device according to claim 1, further comprising:
an alert determination unit configured to perform a statistical process on a basis of a determination result stored in the storage unit, and to determine whether to perform alert to an external device, in accordance with a calculation result.

11. The information processing device according to claim 10, further comprising:
a transmission unit configured to transmit alert to the external device in a case where the calculation result satisfies a predetermined condition.

12. The information processing device according to claim 10, wherein the alert is alert regarding a dementia sign of an elderly person.

13. An information processing method comprising, by a processor:
acquiring a response of a user to a question regarding personal information or action information of the user;
determining true or false of the response; and
storing, into a storage unit, the question, the response, and a determination result in association with each other.

14. A program for causing a computer to function as:
an acquisition unit configured to acquire a response of a user to a question regarding personal information or action information of the user;
a determination unit configured to determine true or false of the response; and
a storage unit configured to store the question, the response, and a determination result in association with each other.
